# FASCICULE DE BREVET EUROPEEN

(11) **EP 3 830 548 B1**
(45) Date de publication et mention de la délivrance du brevet: **05.07.2023**
(21) Numéro de dépôt: 19759662.0
(22) Date de dépôt: 29.07.2019
(51) Int. Cl.: G01N 15/14, G01N 21/64, G01N 15/10, G01N 33/50, G01N 33/86, G01N 21/47

(54) **PROCEDE D'ANALYSE DES PLAQUETTES D'UN ECHANTILLON SANGUIN**
VERFAHREN ZUR ANALYSE VON THROMBOCYTEN EINER BLUTPROBE
METHOD FOR ANALYZING PLATELETS OF A BLOOD SAMPLE

(30) Priorité: 30.07.2018 FR 1857095
(43) Date de publication de la demande: 09.06.2021
(73) Titulaire: Etablissement Français du Sang, 93210 Saint Denis (FR); INSTITUT NATIONAL DE LA SANTE ET DE LA RECHERCHE MEDICALE (INSERM), 75654 Paris Cédex 13 (FR); UNIVERSITE DE STRASBOURG, 67000 Strasbourg (FR)
(72) Inventeur: MAITRE, Blandine, 67000 Strasbourg (FR); ANGENIEUX, Catherine, 67100 Strasbourg (FR); DE LA SALLE, Henri, 67200 Strasbourg (FR); GACHET, Christian, 67220 Lalaye (FR)
(74) Mandataire: Cabinet Beau de Loménie
(86) Numéro de dépôt international: PCT/FR2019/051867
(87) Numéro de publication internationale: WO 2020/025891

(56) Documents cités:
- WO-A1-2009/058876
- Chapman, Lesley M et al: "Platelets present antigen in the context of MHC class I.", PubMed Central (PMC) Author Manuscript THE JOURNAL OF IMMUNOLOGY, 15 juillet 2012 (2012-07-15), pages 1-17, XP055583680, Extrait de l'Internet: URL:https://www.ncbi.nlm.nih.gov/pmc/artic les/PMC3392496/ [extrait le 2019-04-26]
- Abcam: "Product datasheet Anti-HLA Class I antibody [W6/32]", , 12 avril 2006 (2006-04-12), XP055583711, Extrait de l'Internet: URL:https://www.abcam.com/hla-class-i-anti body-w632-ab22432.html [extrait le 2019-04-26]
- BINITA SHAH ET AL: "Mean platelet volume reproducibility and association with platelet activity and anti-platelet therapy", PLATELETS (LONDON), vol. 25, no. 3, 20 juin 2013 (2013-06-20), pages 188-192, XP055583721, GB ISSN: 0953-7104, DOI: 10.3109/09537104.2013.793794
- Anonymous: "Cell surface expression of HLA I molecules as a marker of young platelets - Angénieux - 2019 - Journal of Thrombosis and Haemostasis - Wiley Online Library", , 17 juin 2019 (2019-06-17), XP055647140, Extrait de l'Internet: URL:https://onlinelibrary.wiley.com/doi/ep df/10.1111/jth.14537 [extrait le 2019-11-27]

## Description

### DOMAINE DE L'INVENTION

La présente invention concerne le domaine des procédés d'analyse sanguine *in vitro* et en particulier le domaine des procédés pour analyser des plaquettes présentes dans un échantillon sanguin. Plus particulièrement, le procédé selon l'invention permet de diagnostiquer *in vitro* une thrombopénie périphérique ou centrale chez un sujet.

### ARRIERE-PLAN TECHNIQUE

Les plaquettes, sont des cellules sanguines dépourvues de noyau produites par fragmentation des mégacaryocytes de la moelle osseuse. Elles sont normalement présentes dans le sang à une concentration moyenne de 150 000 à 400 000 /µL chez l'homme et ont un rôle essentiel dans l'hémostase, un processus biologique regroupant l'ensemble des phénomènes qui permettent la prévention et l'arrêt de saignements.

De nombreuses pathologies sont liées à des anomalies du nombre de plaquettes dans le sang. Il peut s'agir d'une augmentation de la concentration de plaquettes dans le sang, c'est-à-dire une thrombocytose, ou d'une diminution de la concentration de plaquettes dans le sang, c'est-à-dire une thrombopénie.

Les thrombocytoses essentielles résultent d'atteintes graves de la moelle osseuse (syndromes myéloprolifératifs en particulier). Détecter ces types de thrombocytoses est primordial pour pouvoir adopter un traitement médical approprié. Une première analyse des plaquettes sanguines et du pourcentage de plaquettes jeunes constitue un geste simple qui permet d'orienter facilement les démarches exploratoires, qui peuvent inclure une analyse de biopsie de moelle, ce qui constitue un geste beaucoup plus invasif.

On peut distinguer deux types de thrombopénies :
- La thrombopénie centrale, ou essentielle, qui résulte d'un défaut, en qualité et/ou en nombre, dans la formation des mégacaryocytes et qui se traduit par une faible production de plaquettes et par conséquent par une faible concentration de plaquettes dans le sang. La thrombopénie centrale peut avoir différentes causes qui peuvent être d'origine génétique, ou acquise comme une carence en vitamine B12, certains cancers, les myélodysplasies, des atteintes hépatiques, ou certains traitements comme la chimiothérapie ou la radiothérapie. Dans certains cas, la cause n'est pas identifiée. Certaines maladies sont connues pour être liées à une thrombopénie centrale, par exemple le syndrome de Bernard-Soulier ou le syndrome de May-Hegglin (MYH9).
- La thrombopénie périphérique se caractérise par une diminution de la numération des plaquettes circulantes, par une augmentation compensatrice de la mégacaryocytopoïèse et donc par une production plus importante de plaquettes. La thrombopénie périphérique est généralement provoquée par la destruction des plaquettes circulantes, par exemple sous l'action d'auto-anticorps ou d'anticorps qui se fixent sur les plaquettes en présence de certains médicaments (héparines, quinines par exemple). Une thrombopénie périphérique se caractérise donc par une proportion plus importante de plaquettes jeunes (parfois dénommées plaquettes immatures). Le purpura thrombopénique idiopathique (PTI ou ITP en anglais), d'origine auto-immune, est la cause la plus fréquente de thrombopénie périphérique. La thrombopénie périphérique peut également être expliquée par un état inflammatoire du patient qui peut entrainer une consommation des plaquettes circulantes ou encore être d'origine allergique.

La thrombopénie peut entraîner des hémorragies, visibles sous forme de pétéchies ou purpura, des saignements anormaux au niveau du nez et de la bouche, des règles abondantes, des hématomes peuvent également survenir.

Le traitement de la thrombopénie dépend de sa cause. Il est donc essentiel de pouvoir distinguer une thrombopénie centrale d'une thrombopénie périphérique.

La thrombopénie est identifiée en mesurant le nombre de plaquettes dans le sang, généralement au moyen d'un test sanguin, appelé « numération formule sanguine » ou « NFS » ou « hémogramme ». La thrombopénie est diagnostiquée si le nombre de plaquettes est inférieur à 150 ×10⁹/L de sang. Néanmoins, la mesure du nombre de plaquettes à elle seule ne permet pas de faire la différence entre une thrombopénie centrale et périphérique.

La détermination de la quantité de plaquettes jeunes dans le sang, c'est-à-dire les plaquettes qui viennent d'être produites par la moelle osseuse, revêt donc un intérêt particulier pour distinguer une thrombopénie centrale d'une thrombopénie périphérique. Les plaquettes jeunes se distinguent des autres plaquettes circulantes par un contenu en ARN de grande taille (ARN messager et ribosomal) plus important et par une taille un peu plus importante. Le thiazole orange (TO) ou ses analogues (ex. l'acridine orange), des. colorants dont l'émission de fluorescence est multipliée par 1000 après liaison à l'ARN, sont utilisés pour discriminer par cytométrie en flux les plaquettes jeunes. Cependant, l'utilisation du TO et de ses analogues pour l'analyse des plaquettes jeunes est délicate, notamment chez l'Homme où la population de plaquettes jeunes ne représente généralement que 1 à 2% de l'ensemble des plaquettes. En effet, une partie de la fluorescence du TO (ou de ses analogues) dans les plaquettes est insensible à un traitement par la RNase qui cible les ARN cytosoliques, ce qui peut s'expliquer par une fluorescence après liaison aux acides nucléiques mitochondriaux ou aux nucléotides présents dans les granules denses. Ainsi, un marquage des plaquettes au TO (ou à un de ses analogues) révèle deux populations : une première dite « TO^{bright} », dont la forte fluorescence est réduite de façon drastique par un traitement avec de la RNase, avec donc un contenu en ARN plus important et qui correspond effectivement aux plaquettes jeunes, une seconde dite « TO^{dim} », dont la fluorescence du TO (ou de ses analogues) est plus faible et est insensible à un traitement avec de la RNase, et qui ne contient donc peu ou pas d'ARN cytosolique et qui ne correspond pas aux plaquettes jeunes.

Malgré cette limite, le procédé utilisé à ce jour pour identifier la cause d'une thrombopénie (i.e. centrale ou périphérique) repose sur l'utilisation d'un analogue du TO pour déterminer la proportion de plaquettes jeunes, appelée « immature platelet fraction » ou « IPF », dans un échantillon de sang total. Il s'agit par exemple du procédé connu sous le nom de Sysmex^{®}. Le procédé Sysmex^{®} utilise un algorithme intégrant la taille des plaquettes et leur fluorescence en présence d'un dérivé de l'acridine orange, une molécule qui possède des propriétés analogues à celles du TO vis-à-vis des ARN. Ce procédé est utilisé pour obtenir un pourcentage d'IPF qui peut être utilisé pour poser un diagnostic clinique face à une thrombopénie isolée. La mise en évidence d'un taux d'IPF élevé est considérée comme reflétant une thrombopénie périphérique alors qu'un taux d'IPF classique mesuré par le procédé Sysmex (inférieur à 15%) peut refléter une thrombopénie centrale. Or, comme expliqué plus haut, une partie de l'acridine orange se lie aux acides nucléiques mitochondriaux ou aux nucléotides dans les granules denses, ces derniers étant insensibles au traitement à la RNase, ce qui peut générer des faux positifs.

Il existe donc un réel besoin de disposer d'un procédé facile à mettre en oeuvre qui peut s'affranchir de l'utilisation du thiazole orange (TO) ou d'un de ses analogues (ex. l'acridine orange) pour pouvoir analyser les plaquettes présentes dans un échantillon sanguin de manière fiable et reproductible, en particulier pour diagnostiquer *in vitro* une thrombopénie périphérique ou une thrombopénie centrale.

L'état de la technique pertinent comprend

Chapman, Lesley M et al: "Platelets present antigen in the context of MHC class I.", PubMed Central (PMC) Author Manuscript THE JOURNAL OF IMMUNOLOGY, 15 juillet 2012 (2012-07-15), pages 1-17, et la publication WO2009/058876A1.

### RESUME DE L'INVENTION

Les inventeurs ont mis en évidence que la mesure par cytométrie en flux du niveau d'expression des molécules du Complexe Majeur d'Histocompatibilité de classe I (CMH I) au niveau de la membrane plasmique des plaquettes est corrélé avec l'âge des plaquettes, un niveau d'expression plus important correspondant à des « plaquettes jeunes ». Ainsi, les inventeurs ont mis au point un procédé permettant d'analyser les plaquettes d'un échantillon sanguin par cytométrie de flux, en utilisant un ligand anti-chaîne alpha du CMH-I couplé à un fluorochrome. Ce procédé d'analyse est particulièrement adapté pour distinguer une thrombopénie périphérique d'une thrombopénie centrale.

L'invention est définie dans les revendications.

### DESCRIPTION DES FIGURES

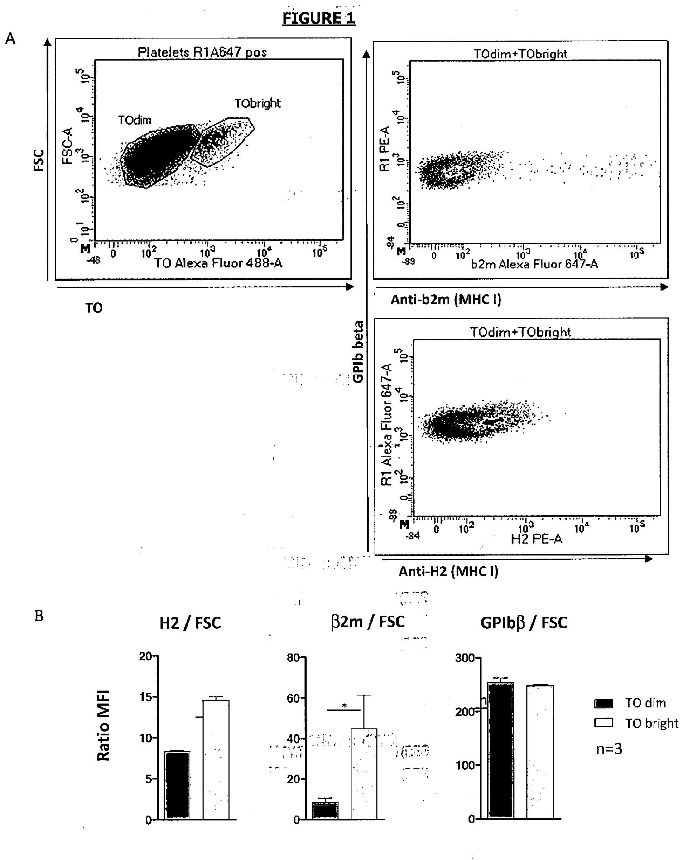

**Figure 1****. L'expression en surface des molécules du CMH I est plus importante sur les plaquettes murines jeunes** A) Graphique d'une analyse FACS représentative de l'expression du TO et du CMH I sur les plaquettes murines. Les plaquettes ont été marquées avec un anticorps anti-GPIb beta conjugué PE ou Alexa-647 et avec du thiazole orange (TO) (panneau de gauche). Les plaquettes GPIb beta+ ont été sélectionnées et l'expression du CMH I a été analysée grâce à un co-marquage avec un anticorps anti-CMHI conjugué PE ou avec un anticorps anti-β2m révélé avec un anticorps secondaire anti-souris de chèvre conjugué Alexa-647 (panneau de droite). Les plaquettes TO^{bright} sont mises en évidence en gris clair (panneaux gauche et droit) B) L'expression en surface des molécules du CMH I et de la GP1bβ a été évaluée en calculant le rapport entre l'intensité moyenne de fluorescence (MFI) du CMH I ou la MFI de la GPIbβ et la MFI du paramètre FSC sur les plaquettes TO^{dim} ou TO^{bright} (* p<0.05, *** p<0.001, ns p>0.05 ; n=3).

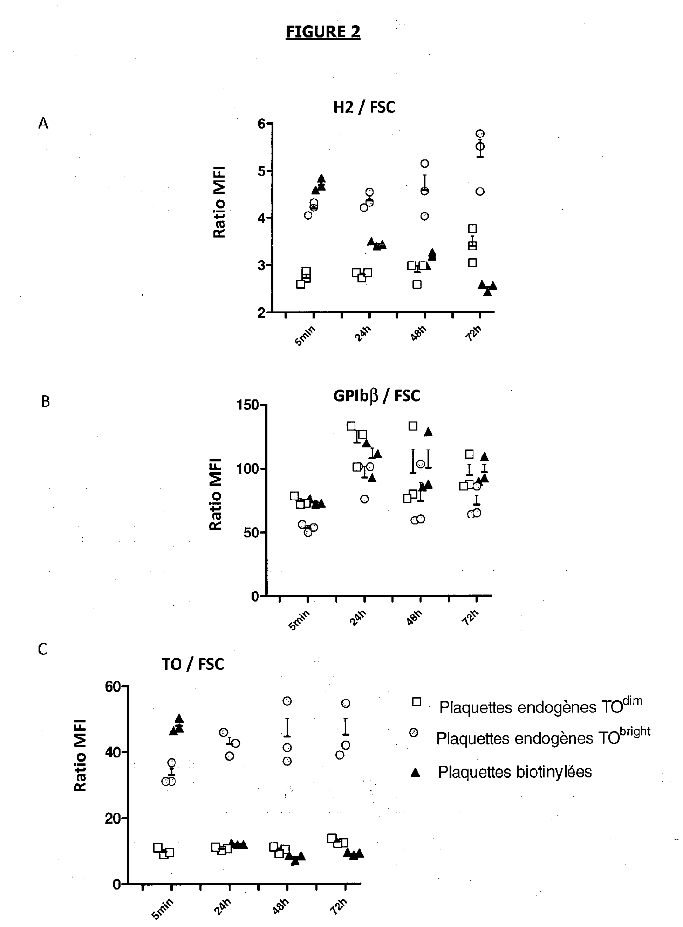

**Figure 2****. L'expression en surface des molécules du CMH I diminue *in vivo* au cours du vieillissement des plaquettes.**
   Des plaquettes de souris iDTR PF4 cre traitées par la toxine diphtérique (DT) ont été isolées, biotinylées et transfusées à des souris WT. Analyse par cytométrie en flux de **A)** l'expression du CMH I **B)** expression de la GPIbβ et **C)** du marquage TO sur plaquettes transfusées biotinylées (▲) et sur plaquettes endogènes TO^{dim} (□) ou TO^{bright} (o) 5 min, 24h, 48h et 72h après transfusion. Résultats exprimés sous forme de ratios entre la MFI du marquage et la MFI du paramètre FSC (n=3, une expérience représentative de 3 expériences indépendantes).

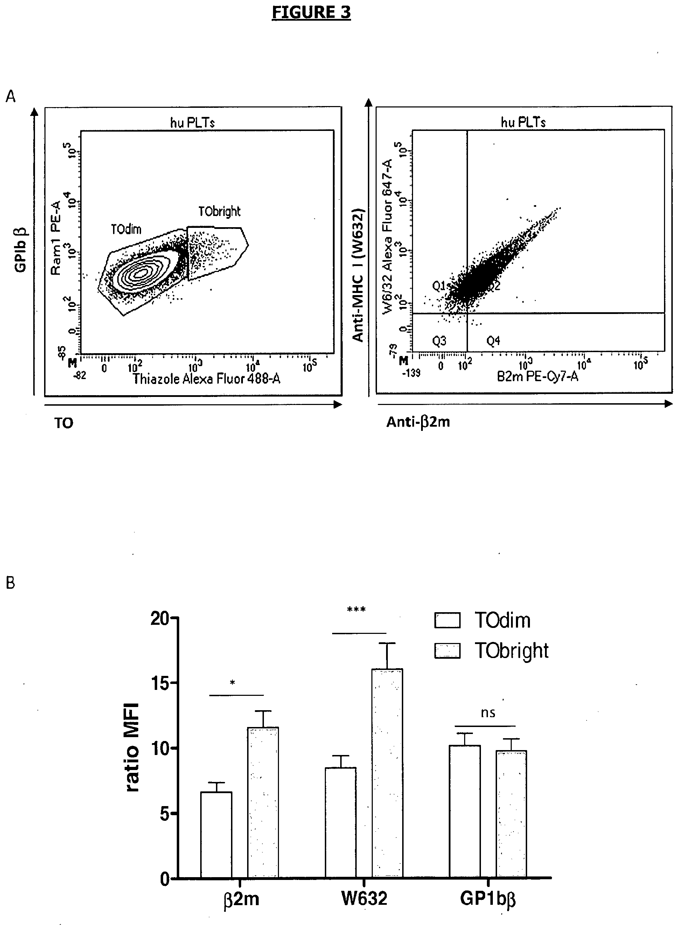

**Figure 3****. Les molécules HLA I sont exprimées de préférence par des plaquettes humaines très fluorescentes pour le thiazole orange A)** Graphiques FACS représentatifs de l'expression du TO et du CMH I sur les plaquettes humaines. Les plaquettes ont été marquées avec un anti-GPIb conjugué PE et avec le TO (panneau de gauche). Les plaquettes GPIbβ+ ont été sélectionnées et l'expression du CMH I a été analysée grâce à une coloration avec un anti-CMHI conjugué A647 et un anticorps anti-β2m conjugué PE-CY7 (panneau de droite). Les plaquettes TO^{bright} sont mises en évidence en gris clair (panneaux de gauche et de droite). **B)** L'expression à la surface cellulaire des molécules du CMH I et de la GP1bβ a été évaluée en calculant le rapport entre la MFI du CMH I (marquage anti-β2m ou anti-HLA I) ou la MFI de la GPIbβ et la MFI du paramètre FSC sur les plaquettes TO^{dim} ou TO^{bright} (^{∗} p<.05, ^{∗∗} p<.01, ns p>.05 ; n=7).

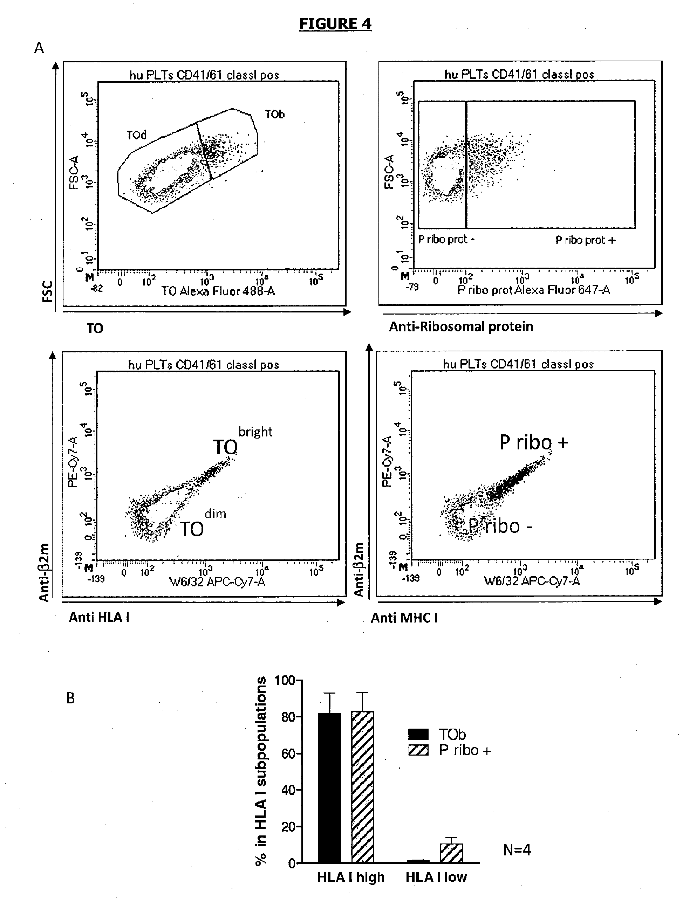

**Figure 4****. Les plaquettes humaines exprimant une densité élevée de molécules du CMH I présentent des caractéristiques de jeunes plaquettes.**
   Graphiques FACS représentatifs de l'expression des protéines ribosomales, du TO et de l'expression des molécules du CMH I sur les plaquettes humaines. Les plaquettes humaines ont été fixées, perméabilisées, co-marquées avec un anti-GPIb conjugué PE, un anticorps anti-protéine ribosomale P mAb, un anti-CMHI conjugué A647, un anticorps anti-β2m conjugué PE-CY7, et le TO puis analysées par FACS. **A)** Les plaquettes GPIbβ+ ont été sélectionnées. L'expression de la protéine ribosomale a été analysée en mettant en évidence les plaquettes TO^{bright} en gris foncé (panneaux du haut à droite). L'expression des molécules du CMH I a été analysée en mettant en évidences les plaquettes TO^{bright} ou P-ribosomal- high en gris foncé (panneaux du bas). **B)** Analyse de la distribution de la protéine ribosomale P ou du marquage TO^{bright} dans les populations exprimant fortement ou faiblement les molécules HLA I.

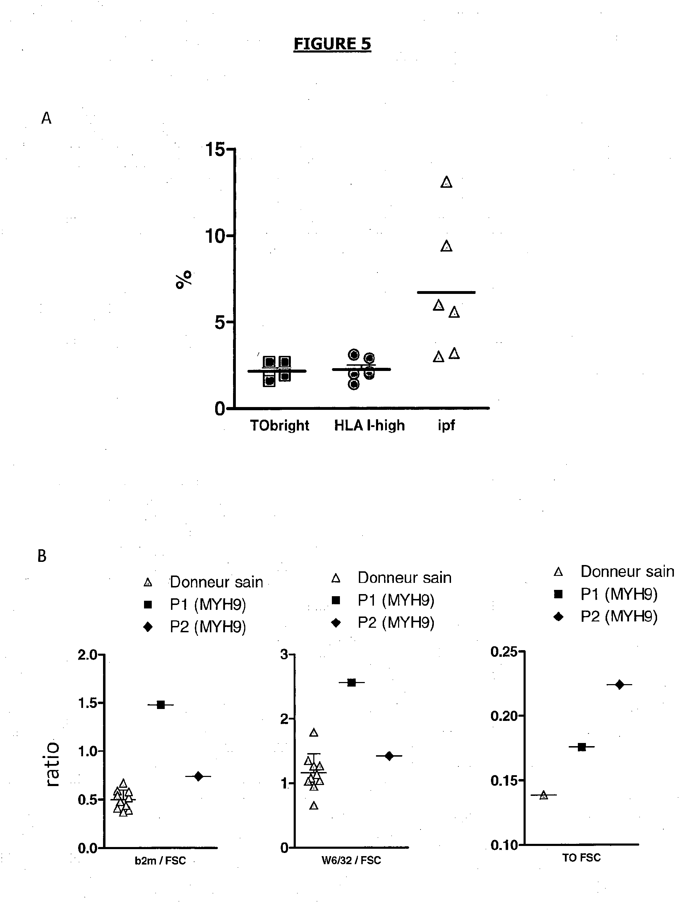

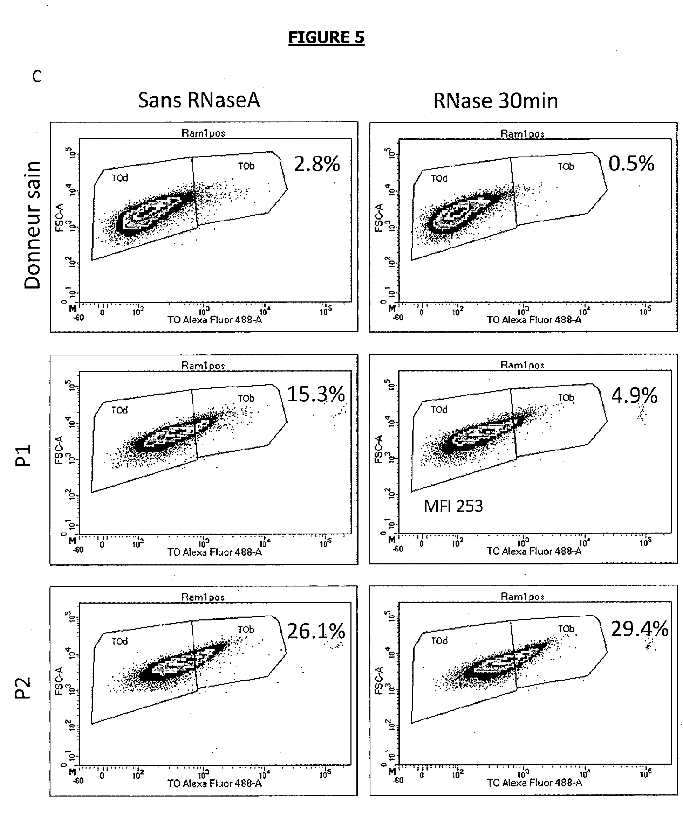

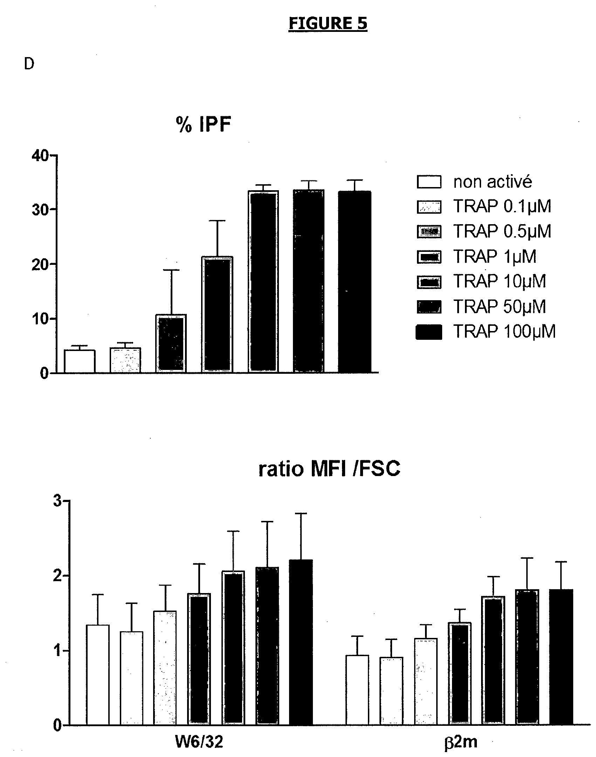

**Figure 5****. L'expression à la surface cellulaire du CMH I est un critère fiable pour déterminer la proportion de jeunes plaquettes chez les patients.**
   A) Sur des échantillons de sang de donneurs sains les pourcentages de plaquettes TO^{bright} et HLA I^{high} ont été déterminés par analyse FACS, et les pourcentages d'IPF avec l'automate Sysmex^{®} (n=6) B) Des échantillons de sang de donneurs sains ou de patients déficients en MyH9 ont été analysés et la MFI du TO et de HLA I sur les plaquettes ont été déterminés par analyse FACS. Les résultats sont exprimés sous forme de ratios entre les MFI des marquages HLA I (W6/32 ou β2m) ou TO sur les MFI des FSC. C) Graphiques de FACS du marquage TO sur les différents échantillons de sang avant et après le traitement à la RNase. Les pourcentages de plaquettes TO^{bright} sont indiqués pour chaque condition. D) Les échantillons de sang citraté de donneur sain ont été activés par du TRAP 0.1, 0.5, 1, 10, 50 ou 100 µM avant que les pourcentages d'IPF soient estimés par le Sysmex^{®} (panneau du haut) ou que les ratios HLA I/FSC ou β2m/FSC soient analysés par cytométrie en flux (panneau du bas) (n=3-5).

### DESCRIPTION DETAILLEE

Les Demandeurs ont mis au point un procédé permettant d'analyser les plaquettes présentes dans un échantillon sanguin de façon précise et reproductible. La mise en oeuvre de ce procédé permet notamment de déterminer *in vitro* l'origine (centrale ou périphérique) d'une thrombopénie.

### Définitions

Les « plaquettes » sont des cellules sanguines produites par fragmentation des mégacaryocytes de la moelle osseuse. Les plaquettes sont dépourvues de noyau chez les mammifères et leur durée de vie chez l'homme est d'environ 7 à 10 jours avant d'être éliminées par les macrophages de la rate ou du foie. Les plaquettes sont des composants indispensables de l'hémostase primaire. Chez l'homme, les plaquettes ont un diamètre de 2 à 4 microns et leur concentration normale dans le sang est de 150 à 400×10⁹ plaquettes par litre de sang. Leur nombre tend à diminuer avec l'âge. Les plaquettes expriment les molécules du CMH de classe I (Complexe Majeur d'histocompatibilité) à leur surface.

Le terme « plaquettes jeunes » désigne les plaquettes nouvellement produites par la moelle osseuse, c'est-à-dire les plaquettes ayant moins de 24 heures, par exemple moins de 12 heures.

Lorsque la concentration en plaquettes est inférieure à 150 ×10⁹ plaquettes par litre de sang, le taux de plaquettes est considéré bas et on parle de « thrombopénie » ou de « thrombocytopénie », à laquelle le risque d'hémorragie est augmenté.

Lorsque la concentration en plaquettes est supérieure à 400 ×10⁹ plaquettes par litre de sang, le taux de plaquettes est considéré haut et on parle alors de « thrombocytose », à laquelle sont associés des risques de thrombose.

On peut distinguer deux types de thrombopénies :
- La thrombopénie centrale, ou essentielle, qui résulte d'un défaut, en qualité et/ou en nombre, dans la formation des mégacaryocytes et qui se traduit par une faible production de plaquettes (concentration en plaquettes dans le sang inférieure à 150 ×10⁹ plaquettes par litre de sang). La thrombopénie centrale peut avoir différentes causes qui peuvent être d'origine génétique, ou acquise comme une carence en vitamine B12, certains cancers, les myélodysplasies, des atteintes hépatiques, ou certains traitements comme la chimiothérapie ou la radiothérapie. Dans certains cas, la cause n'est pas identifiée. Certaines maladies sont connues pour être liées à une thrombopénie centrale, par exemple le syndrome de Bernard-Soulier ou le syndrome de May-Hegglin (MYH9).
- La thrombopénie périphérique se caractérise par une diminution de la numération des plaquettes circulantes, par une augmentation compensatrice de la mégacaryocytopoïèse et donc par une production plus importante de plaquettes. La thrombopénie périphérique est généralement provoquée par leur destruction, par exemple sous l'action d'auto-anticorps ou d'anticorps qui se fixent sur les plaquettes en présence de certains médicaments (héparines, quinines par exemple). Une thrombopénie périphérique se caractérise donc par une proportion plus importante de plaquettes jeunes (parfois dénommées plaquettes immatures). Le purpura thrombopénique idiopathique (PTI ou ITP en anglais), d'origine auto-immune, est une cause fréquente de thrombopénie périphérique. La thrombopénie périphérique peut également être expliquée par un état inflammatoire du patient qui peut entraîner une consommation des plaquettes circulantes ou être d'origine immune.

Les molécules du « Complexe Majeur d'Histocompatibilité » de classe I ou « CMH I » constituent le système de reconnaissance du soi et présentent des antigènes peptidiques aux lymphocytes T. Chez l'homme le CMH I est appelé « HLA de classe I » pour « Human Leucocyte Antigen de classe I ». Ces molécules sont exprimées à la membrane plasmique de presque toutes les cellules nucléées et sur les plaquettes sanguines. Elles sont constituées de l'association non covalente d'une chaîne alpha (comprenant 3 domaines de type immunoglobuline : a1, a2 et a3) et de la béta-2 microglobuline (β2m). Le CMH I exprimé à la membrane plasmique est toujours associé à un peptide, lequel permet de stabiliser le CMH I. Le CMH I est notamment impliqué dans la présentation des antigènes aux lymphocytes T CD8, permettant de discriminer le soi du non soi.

Le terme « échantillon sanguin », tel qu'utilisé ici, comprend tout échantillon contenant des plaquettes. L'échantillon sanguin est par exemple obtenu à partir du sang d'un sujet. De préférence, il s'agit d'un échantillon de sang total, avantageusement prélevé en présence d'anticoagulant selon les bonnes pratiques de l'Homme de l'art. L'anticoagulant peut être l'EDTA ou le citrate.

Le terme « ligand » au sens de l'invention désigne une molécule qui se lie de façon spécifique à la cible. Par exemple, dans le cadre de la présente invention, un ligand qui se lie au CMH I désigne donc un ligand qui se lie de façon spécifique au CMH I.

Dans un mode de réalisation particulier de l'invention, le ligand n'est pas capable de se lier à la chaîne β2m non associée à la chaîne alpha, c'est-à-dire à la chaîne β2m soluble.

Dans un autre mode de réalisation particulier de l'invention, le ligand est capable de se lier à la chaîne β2m non associée à la chaîne alpha. Dans ce mode de réalisation particulier, une concentration de ligand suffisamment élevée est avantageusement utilisée pour que la chaîne β2m soluble présente dans le l'échantillon sanguin n'interfère pas avec la liaison au CMH I lors de la mise en oeuvre du procédé selon l'invention.

Dans un mode de réalisation particulier de l'invention, le ligand est capable de se lier à tous les isotypes de CMH I, c'est à dire les isotypes A (HLA A), B (HLA B) et C (HLA C).

Le ligand peut être un anticorps, un fragment d'anticorps, un aptamère, une protéine, un peptide ou une petite molécule. Les ligands utilisables dans le cadre de l'invention peuvent être obtenus facilement par l'Homme de l'art. On peut citer par exemple le procédé Selex pour les aptamères ou les procédés d'immunisation et de clonage pour les anticorps. Dans un mode de réalisation préféré, le ligand est un anticorps ou un fragment d'anticorps. Plusieurs anticorps utilisables dans le cadre de l'invention sont accessibles dans le commerce, par exemple l'anticorps W6/32, produit par l'hybridome ATCC HB-95, par exemple l'anticorps W6/32 déjà couplé à l'APC disponible chez Biolegend, ou l'anticorps anti-β2m humaine déjà couplé au fluorophore PE/Cy7 disponible chez Biolegend (référence : 316318).

Le terme « anticorps » est utilisé ici dans son sens le plus large et englobe diverses structures d'anticorps qui présentent l'activité de liaison à l'antigène souhaité, notamment les anticorps monoclonaux. Il peut s'agir d'anticorps humains, humanisés, chimériques ou d'une espèce non-humaine, par exemple des anticorps murins.

Dans le cadre de la présente invention, le ligand est couplé à un moyen permettant sa détection en cytométrie de flux, de préférence un fluorochrome (ou fluorophore). Le terme « fluorochrome » désigne une molécule capable d'émettre de la lumière de fluorescence après excitation. L'intensité de fluorescence (ou MFI) émise par le fluorochrome peut être mesurée par tout moyen connu, par exemple avec un cytomètre de flux capable de détecter et de mesurer l'intensité de fluorescence du fluorochrome.

La « cytométrie en flux » ou « cytométrie de flux » est une technique largement répandue qui permet de faire défiler des particules ou cellules à grande vitesse à travers le faisceau d'un laser, en les comptant et en les caractérisant. La cytométrie de flux est une technique particulièrement avantageuse car elle permet de mesurer individuellement l'intensité de fluorescence des particules, molécules ou cellules en les faisant défiler une par une devant un faisceau laser.

Le « standard interne » au sens de l'invention permet de normaliser les intensités de fluorescence (MFI) mesurées en cytométrie de flux. Dans le cadre de la présente invention, le standard interne est de préférence un élément qui se lie au ligand. Il peut notamment s'agir d'une microbille de polystyrène sur laquelle peut s'adsorber le ligand. De nombreux standards internes adaptés à différents types de ligands sont proposés dans le commerce, par exemple le standard « BD Compbead n°552843 » qui peut être utilisé lorsque le ligand est un anticorps qui comprend la chaine kappa d'un anticorps de souris. Par « sujet » au sens de l'invention, il est fait référence à un mammifère, par exemple un chien, un chat, un ovin, un bovin ou l'Homme, de préférence le sujet est un Homme. Par « sujet sain » au sens de l'invention, il est fait référence à un sujet qui ne présente aucune pathologie à l'origine d'une numération anormale des plaquettes dans le sang et qui n'est pas sous traitement médicamenteux.

Le terme « diagnostic », est utilisé ici dans son sens le plus large, et est couramment utilisé et bien compris de l'Homme de l'art. Ce terme désigne par exemple la détermination de la probabilité, du risque ou de la possibilité d'avoir une pathologie chez un sujet.

Le terme « traitement » ou « traiter » ou « répondre au traitement » désigne au sens de l'invention une amélioration, une atténuation, une inversion ou une inhibition de la progression d'une pathologie. En particulier, le traitement de la pathologie peut consister à rétablir une production normale de plaquettes par la moelle osseuse.

### Procédé pour analyser les plaquettes ou identifier une population de plaquettes

Les demandeurs se sont en effet aperçus que le niveau d'expression du CMH I à la surface des plaquettes est corrélé avec leur âge. Ainsi, les plaquettes les plus jeunes expriment plus de CMH I au niveau de leur membrane plasmique que les plaquettes plus âgées.

En particulier, les demandeurs se sont en effet aperçus que le niveau d'expression du CMH I à la surface des plaquettes normalisé par rapport au paramètre FSC est corrélé avec leur âge. Ainsi, les plaquettes les plus jeunes ont une densité de molécules de CMH I au niveau de leur membrane plasmique plus importante que les plaquettes plus âgées.

### Etape a)

L'étape a) consiste à ajouter audit échantillon un ligand qui se lie au CMH I couplé à un fluorochrome. Cette étape est réalisée en ajoutant le ligand couplé à un fluorochrome dans un échantillon sanguin, par exemple sous agitation. L'agitation permet notamment d'homogénéiser le mélange afin de faciliter le contact entre les ligands marqués et les plaquettes.

Le temps d'incubation doit être suffisant pour mettre en contact le ligand marqué et les plaquettes. Généralement, le temps d'incubation utilisé par l'Homme du métier est de 20 à 30 min pour un anticorps, mais il pourrait être raccourci en fonction du ligand utilisé et être alors inférieur à 5 min, par exemple inférieur à 4 min, inférieur à 3 min, inférieur à 2 min, inférieur à 1 minute, inférieur à 30 secondes. Le temps d'incubation peut également être supérieur à 5 min, supérieur à 10 min ou même supérieur à 15 min.

Le procédé d'analyse selon l'invention est particulièrement facile à mettre en oeuvre puisqu'il peut être mis en oeuvre en utilisant un échantillon de sang total. Néanmoins, il n'est pas limité à l'utilisation d'un échantillon sanguin spécifique. Par exemple, il peut s'agir de toute fraction du sang total comprenant des plaquettes, par exemple le plasma sanguin riche en plaquettes (PRP) ou des plaquettes isolées à partir de sang total. De préférence, l'échantillon sanguin est un échantillon de sang total.

Dans un mode de réalisation particulier, l'échantillon sanguin peut contenir d'autres composants que ceux naturellement présents dans l'échantillon sanguin, par exemple des tampons, qui permettent d'ajuster le pH, ou des agents anticoagulants adaptés tels que l'EDTA ou le citrate.

Dans un mode de réalisation particulier, le ligand peut être choisi parmi un anticorps, un fragment d'anticorps, un aptamère, une protéine, un peptide ou une petite molécule. La quantité de ligand appropriée peut être déterminée aisément par l'expérimentateur. Par exemple, si le ligand est un anticorps, il peut être ajouté à une concentration allant de 0,1 µg/mL à 10 µg/mL. Le ligand est ajouté à l'échantillon en condition saturante afin d'assurer un marquage approprié des plaquettes.

Dans un mode de réalisation particulier, le ligand est un anticorps ou un fragment d'anticorps, par exemple l'anticorps W6/32 produit par l'hybridome ATCC HB-95 ou un fragment qui a gardé sa capacité à se lier au CMH I. Il peut également s'agir d'un anticorps qui se lie à la β2m, dans ce cas il faut utiliser une concentration d'anticorps suffisamment élevée pour que la β2m soluble présente dans le sang n'interfère pas lors de la mise en oeuvre de la méthode.

Le ligand est couplé à un moyen permettant sa détection avec un cytomètre en flux, de préférence un fluorochrome (ou fluorophore). Le ligand peut être couplé à un fluorochrome directement ou indirectement, de préférence directement. Des méthodes de couplage d'un ligand avec un fluorochrome sont largement décrites dans la littérature. Le couplage ne présente donc aucune difficulté technique particulière.

Le procédé n'est pas limité à un fluorochrome particulier, à partir du moment où il peut être détecté avec un cytomètre en flux. Avantageusement, le fluorochrome est un fluorochrome dont les longueurs d'onde d'excitation et d'émission n'interfèrent pas avec les réactifs permettant de détecter l'ARN. Des exemples, non limitatifs, de fluorophores sont cités ci-après et dans les exemples : FITC (fluorescein isothiocyanate), PE (R-phycoerythrin), APC (allophycocyanin), PerCP (peridinin chlorophyll protein), PE-CF594, peridinin chlorophyll protein (PerCP)-Cy5.5, PE-Texas Red, GFP (green fluorescent protein), YFP (yellow fluorescent protein) ou CFP (cyan fluorescent protein).

### Etape b)

L'étape b) consiste à mesurer l'intensité de fluorescence moyenne des plaquettes (MFI_{plaquettes}) avec un cytomètre en flux. Bien entendu, le cytomètre en flux est capable de détecter et de mesurer la fluorescence du fluorochrome. La mesure est effectuée automatiquement par le cytomètre en flux. Le paramétrage du cytomètre en flux ne présente aucune difficulté technique particulière. En particulier, l'Homme du métier saura paramétrer le cytomètre en flux en fonction du fluorochrome choisi.

La mesure effectuée à l'étape b) permet d'identifier les plaquettes liées par le ligand marqué, et permet l'identification d'une ou de plusieurs population(s) de plaquette(s), en particulier une population de plaquettes jeunes.

### Etape c)

L'étape c) consiste à mesurer le paramètre « Forward Scatter » moyen (FSC) avec ledit cytomètre en flux et déterminer le ratio MFI_{plaquettes} / FSC. En plus de l'intensité de fluorescence, le cytomètre en flux peut mesurer d'autres paramètres qui permettent notamment de donner une indication sur la taille des particules, par exemple sur la taille des plaquettes. Le paramètre le plus couramment mesuré pour donner une indication sur la taille des plaquettes est le « Forward Scatter » (FSC), également appelé ci-après « FSC_{plaquettes} ». Il peut s'agir du FSC-A, FSC-H ou FCS-W. Le FSC-A est généralement utilisé.

Dans un mode de réalisation particulier, la mesure MFI_{plaquettes} est normalisée avec l'intensité de fluorescence moyenne d'un standard interne (MFI_{standard}) afin d'obtenir une intensité de fluorescence moyenne des plaquettes normalisée (MFI_{plaquettes normalisée}). Le standard interne est de préférence ajouté audit échantillon à l'étape a), soit avant le ligand soit après le ligand, soit simultanément au ligand, de préférence avant le ligand. La quantité de standard interne appropriée peut être déterminée aisément par l'expérimentateur, de préférence en le mettant en quantité d'un même ordre de grandeur que le nombre de plaquettes.

La normalisation de la mesure MFI_{plaquettes} avec un standard interne est particulièrement avantageuse puisqu'elle permet de palier la variabilité inter-expérience, inter-laboratoire et/ou inter-expérimentateur.

Dans un mode de réalisation particulier, le standard interne se lie au ligand couplé au fluorochrome, par exemple le ligand couplé au fluorochrome s'adsorbe sur le standard interne. Dans ce mode de réalisation particulier, la MFI_{standard} correspond donc à l'intensité de fluorescence moyenne émise par le fluorochrome lié au standard et mesurée avec le cytomètre en flux capable de détecter et de mesurer la fluorescence du fluorochrome. Le cytomètre en flux doit être réglé pour faire la distinction entre les plaquettes marquées et le standard interne marqué. En général, la distinction se fait par la taille. Lorsque la distinction se fait par la taille, il est donc essentiel que la taille du standard marqué soit choisie de façon à ce que le standard se démarque aisément des plaquettes.

Avantageusement, le standard interne est une microbille de polystyrène sur lequel s'adsorbe le ligand couplé au fluorochrome. Il peut s'agir, par exemple, d'une microbille de polystyrène recouverte d'anticorps qui se lient audit ligand couplé au fluorochrome. On peut citer par exemple le produit commercial « BD Compbead n°552843 » qui peut être utilisé lorsque le ligand est un anticorps qui comprend la chaîne kappa d'un anticorps de souris (ex. un anticorps chimérique).

Dans un mode de réalisation préféré, le procédé de l'invention comprend en outre une étape c) mesurer le paramètre de diffusion FSC moyen (FSC) avec ledit cytomètre en flux et déterminer le ratio MFI_{plaquettes} / FSC ou MFI_{plaquettes normalisée} / FSC. La détermination du ratio MFI_{plaquettes} / FSC ou MFI_{plaquettes normalisée} / FSC est particulièrement avantageuse puisqu'elle permet d'analyser plus finement les plaquettes. Cela permet de différencier plus finement une ou plusieurs population(s) de plaquette(s), en particulier une population de plaquettes jeunes. L'étape c) est effectuée automatiquement par le cytomètre en flux simultanément à l'étape b). Dans ce mode de réalisation particulier, la détermination des ratios peut être faite automatiquement avec les valeurs mesurées par le cytomètre en flux. Le ratio MFI_{plaquettes normalisée} / FSC peut, par exemple, être obtenu en calculant le ratio (MFI _{plaquettes} / FSC_{plaquettes}) / (MFI _{standard} / FSC_{standard}). Comme pour FSC_{plaquettes} qui donne une indication sur la taille des plaquettes, le paramètre FSC_{standard} donne une indication sur la taille des particules de standard.

L'invention concerne également un procédé pour identifier une population de plaquettes, de préférence une population de plaquettes jeunes, présente dans un échantillon sanguin, ledit procédé comprend les étapes de :
a) analyser les plaquettes présentes dans un échantillon sanguin par la mise en oeuvre du procédé selon l'invention ; et
b) utiliser le résultat de l'étape a) pour identifier une population de plaquettes, de préférence une population de plaquettes jeunes.

L'identification de la population de plaquettes est faite automatiquement par le cytomètre de flux en fonction des paramètres décrits précédemment.

### Procédé de diagnostic

L'invention concerne un procédé de diagnostic *in vitro* d'une thrombopénie périphérique ou centrale chez un sujet comprenant les étapes de :
a) analyser les plaquettes présentes dans un échantillon sanguin d'un sujet en mettant en oeuvre le procédé d'analyse des plaquettes selon l'invention ; et
b) utiliser le résultat de l'étape a) dans le diagnostic de la thrombopénie périphérique ou centrale.

Le résultat obtenu en analysant les plaquettes selon l'invention permet de poser un diagnostic de la thrombopénie périphérique chez un sujet. En particulier, un diagnostic positif pour la thrombopénie périphérique est donné par une augmentation chez ledit sujet du/des paramètre(s) MFI_{plaquettes}, de MFI_{plaquettes normalisée}, du ratio MFI_{plaquettes} / FSC et/ou du ratio MFI_{plaquettes normalisée} / FSC par rapport au(x) même(s) paramètre(s) mesuré(s) chez un sujet sain.

Une augmentation du ou des paramètre(s) précité(s) chez le sujet considéré en comparaison avec ce ou ces même(s) paramètres(s) mesuré(s) chez un sujet sain, traduit une augmentation de la quantité de plaquettes jeunes chez ledit sujet considéré et permet de mettre en évidence une activité accrue de la moelle osseuse et donc permet de poser le diagnostic d'une thrombopénie périphérique.

Dans un mode de réalisation préféré, l'augmentation d'un ou de plusieurs des paramètres précités est de l'ordre d'un facteur supérieur à 1,5, par exemple supérieur à 2, supérieur à 2,5, supérieur à 3, ou encore supérieur à 3,5.

Le résultat obtenu en analysant les plaquettes selon l'invention permet de poser un diagnostic de la thrombopénie centrale chez un sujet. En particulier, un diagnostic positif pour la thrombopénie centrale est donné par un/des paramètre(s) MFI_{plaquettes}, MFI_{plaquettes normalisée}, ratio MFI_{plaquettes} / FSC et/ou ratio MFI_{plaquettes normalisée} / FSC chez ledit sujet similaire(s) ou identique(s) au(x) même(s) paramètre(s) mesuré(s) chez un sujet sain. Dans un mode de réalisation particulier, ledit sujet a préalablement été diagnostiqué comme présentant une thrombopénie ou comme étant susceptible de présenter une thrombopénie. Le procédé de diagnostic selon l'invention va alors permettre de connaître l'origine (centrale ou périphérique) de la thrombopénie.

### Procédé pour suivre l'efficacité thérapeutique d'un traitement

L'invention concerne également un procédé *in vitro* de suivi de l'efficacité thérapeutique d'un traitement de la thrombopénie centrale ou périphérique, comprenant les étapes de :
a) analyser les plaquettes présentes dans un échantillon sanguin d'un patient ayant subi un traitement de la thrombopénie centrale ou périphérique en mettant en oeuvre le procédé d'analyse selon l'invention ;
b) utiliser le résultat de l'étape a) dans la détermination de l'efficacité thérapeutique du traitement de la thrombopénie centrale ou périphérique.

### Procédé de pronostic

L'invention concerne également un procédé *in vitro* pour pronostiquer le développement d'une maladie inflammatoire ou cardiovasculaire, comprenant les étapes de :
a) analyser les plaquettes présentes dans un échantillon sanguin d'un sujet en mettant en oeuvre le procédé d'analyse selon l'invention ;
b) utiliser le résultat de l'étape a) dans le pronostic du développement de la maladie inflammatoire ou cardiovasculaire.

Dans un mode de réalisation particulier, la maladie inflammatoire est le sepsis. Dans un autre mode de réalisation particulier, la maladie cardiovasculaire est l'athérosclérose.

### EXEMPLES

### Matériels et méthodes

### Réactifs

Les réactifs sont disponibles dans le commerce. Le thiazole orange (TO) provient de chez Sigma-Aldrich. La xylazine (Rompun^{®}) et la ketamine (Imalgene 1,000^{®}) sont de Bayer et de Merial, respectivement. La biotine est fournie par ThermoFischer et la toxine diphtérique (DT) par Santa Cruz.

### Anticorps et cytométrie en flux

L'anticorps monoclonal de rat dirigé contre le domaine extracellulaire de la glycoprotéine plaquettaire GPIb beta (Ram1, [7]) a été produit et couplé à l'Alexa647 ou 488 dans notre laboratoire. Les anticorps de souris anti-HLAI (clone W6/32) couplé à l'allophycocyanin (APC) (référence : 311410), anti-β2m humaine couplé au PE-CY7 (référence : 316318) et anti-CD45 humain couplé à APC-cy7 (clone 2D1) (référence : 368516) proviennent de chez Biolegend. L'anti-H2 de souris (clone M1/42) couplé à la phycoérythrine (PE) (référence : 125506) et la streptavidine couple à l'APC-CY7 (référence : 405208) ont été achetés chez Biolegend. L'anticorps anti-b2 microglobuline (β2m) de souris (clone S19.8) (référence : 555299) provient de BD Pharmingen. L'anticorps anti-CD45 de souris couplé à la PE-cy7 (clone 30-F11) (référence : 25-0451) a été fourni par Invitrogen. L'anticorps anti-protéine ribosomale P humaine a aimablement été fourni par le Prof. Sun, National Yang-Ming University, Taiwan [1].

Le sang total a été dilué dans 10 volumes de PBS contenant 1% de BSA et 6 mM EDTA. Les récepteurs Fcγ ont été bloqués par le réactif « FcR blocking » (Milteny Biotec) dilué au 1/200^{e} avant le marquage avec le ou les anticorps. Après une étape de lavage, les cellules ont été contre-marquées avec du TO 1µg/ml dans du PBS pendant 15 min. Les échantillons ont ensuite été dilués dans 20 volumes de paraformaldehyde 1% et analysés par cytométrie de flux (LSRFortessa^{™} cell analyzer, BD Biosciences) dans les 45 minutes. Les données ont été analysées avec le logiciel BD FACSDiva.

### Souris

Les expériences ont été réalisées sur des souris C57BI6/J âgées de 8 à 10 semaines achetées chez °Charles River Laboratories". Les souris positives à la fois pour les transgènes du récepteur à la toxine diphtérique et le PF4 - désignées dans le texte par PF4-cre/iDTR - ont été générées comme décrit précédemment [2].

### Transfusion plaquettaire

Les souris PF4-cre/iDTR ont été rendues sévèrement thrombopéniques par injection de toxine diphtérique (DT) (100 ng/jour, i.p.) pendant 4 jours. 8 jours après la dernière injection, le sang a été prélevé à l'aorte abdominale des souris anesthésiées sur citrate (3.8%) utilisé comme anticoagulant et les plaquettes ont été isolées comme décrit précédemment [3]. Les plaquettes réticulées lavées ont été biotinylées, resuspendues à 1.2.10⁶ /µL et 150 pL de la suspension ont été injectées dans le sinus rétro-orbitaire des souris WT. Les échantillons de sang ont été collectés 5 minutes après la transfusion (temps 0) puis 30min, 2h, 24, 48 et 72 h après.

### Déclaration éthique

Les échantillons de sang humain ont été obtenus auprès de donneurs volontaires et non rémunérés, recrutés par l'Etablissement Français du Sang - Grand Est (EFS-Alsace), où la recherche a été effectuée. À chaque collecte de sang, les donneurs ont signé un consentement éclairé indiquant que les échantillons pouvaient être utilisés à des fins de recherche. L'approbation éthique des expériences sur les animaux était conforme à la directive de l'Union européenne 2010/63/EU. L'étude a été approuvée par le Comité régional d'éthique pour l'expérimentation animale de Strasbourg, C.R.E.M.E.A.S. (CEEA 35).

### Analyses statistiques

Les analyses statistiques ont été faites à l'aide du logiciel GraphPad (Prism 5.02).

### Résultats

### Les plaquettes murines jeunes expriment plus de molécules du CMH I à leur surface

Dans un premier temps, nous avons analysé si l'expression des molécules de classe I à la surface des plaquettes de souris dépendait de leur âge. Chez la souris, le marquage au thiazole orange (TO) a permis de discriminer facilement deux populations plaquettaires, environ 10% d'entre elles présentent une fluorescence importante au TO (TO^{bright}) (Figure 1A, gauche) et sont âgées de moins de 12 heures [2]. L'expression des molécules du CMH I à la surface des plaquettes a été estimée par cytométrie de flux après un marquage avec un anticorps anti-β2m ou anti-CMH I (H2) suivi d'un marquage au TO. L'expression des molécules de classe I est apparue plus forte sur les plaquettes TO^{bright} que sur les plaquettes TO^{dim} (Figure 1A, droite). Pour exclure le fait que les plaquettes jeunes puissent exprimer plus de molécules du CMHI de par leur taille plus grande, nous avons calculé le ratio de l'intensité moyenne de fluorescence (MFI) du marquage de la β2m ou de la chaîne lourde (H2) sur la moyenne du paramètre FSC (un paramètre corrélé à la taille des cellules) dans les deux populations TO^{dim} et TO^{bright} (Figure 1B). Le ratio était significativement plus élevé dans la population TO^{bright} que dans la population de plaquettes TO^{dim} (ratio H2/FSC 14.55 +/- 0.46 dans TO^{bright} vs 8.35 +/- 0.14 dans TO^{dim}; p< 0.001 et ratio β2m/FSC 44.78 +/- 16.50 dans TO^{bright} vs 8.25 +/- 2.32 dans TO^{dim}; n=3; p < 0.05). Par contre, lorsque nous avons analysé l'expression de la GPIbβ, une glycoprotéine majeure spécifique des plaquettes, ce ratio était similaire pour les deux populations plaquettaires (ratio GPIb/FSC 247.07 +/- 3.57 dans TO^{bright} vs 254.70 +/-8.15 dans TO^{dim}; n=3; ns).

Dans l'ensemble, ces données ont montré que dans des conditions homéostatiques, les plaquettes murines jeunes expriment plus de molécules du CMH I à leur surface.

### L'expression en surface des molécules de classe I diminue in vivo au cours du vieillissement des plaquettes

Nous avons ensuite déterminé si l'expression de surface du CMH I sur les plaquettes était affectée par leur vieillissement *in vivo.* Nous avons profité des souris iDTR PF4 cre pour obtenir une population de jeunes plaquettes synchrones [2] L'administration quotidienne de la toxine diphtérique (DT) pendant 4 jours a induit l'ablation des mégacaryocytes matures, bloquant la production de plaquettes et entraînant une thrombocytopénie progressive. Quatre jours après l'arrêt du traitement (le 8e jour), la mégacaryopoïèse et la thrombopoïèse ont été considérablement améliorées, ce qui a entraîné la présence transitoire d'une vaste majorité de jeunes plaquettes (données non montrées et [2]). Ainsi, des plaquettes de souris iDTR PF4 cre traitées par la DT ont été isolées le 8e jour, biotinylées et transfusées à des souris WT. L'expression du CMH I sur les plaquettes transfusées biotinylées a été analysée et comparée à l'expression du CMH I sur les plaquettes endogènes par cytométrie de flux à différents moments après la transfusion. Les résultats ont montré que le rapport (fluorescence moyenne de TO)/(FSC moyen) était élevé pour les plaquettes biotinylées 5 min après la transfusion et comparable aux plaquettes TO^{bright} endogènes. 24h plus tard, il était égal à celui des plaquettes endogènes TO^{dim} (Figure 2C), ce qui confirme le vieillissement *in vivo* des plaquettes transfusées. Fait remarquable, l'expression du CMH I sur les plaquettes transfusées biotinylées a diminué graduellement au fil du temps. Cinq minutes après la transfusion, l'expression du CMH I sur les plaquettes biotinylées était similaire au niveau d'expression sur les plaquettes To^{bright} endogènes. Vingt-quatre heures plus tard, ce niveau d'expression a diminué considérablement pour atteindre le même niveau d'expression que sur les plaquettes TO^{dim} endogènes (Figure 2A). Cette expérience montre qu'*in vivo,* le niveau d'expression des molécules du CMH I à la surface des plaquettes est lié à leur âge. Par contre, l'expression GPIbβ était stable sur les plaquettes transfusées sur une période de 72 heures (Figure 2B).

Dans l'ensemble, ces données ont mis en évidence la pertinence du paramètre d'expression du CMH I pour discriminer les jeunes plaquettes de moins de 24h.

### Les molécules HLA I sont préférentiellement exprimées par les plaquettes humaines TO^{bright}

Chez l'Homme, les plaquettes TO^{bright}, représente entre 1 et 2 % de la population totale de plaquettes (Figure 3A, panneau de gauche). Pour caractériser l'expression des molécules HLA I à la surface des plaquettes humaines, ces dernières ont été co-marquées avec un anticorps anti-β2m, un anticorps pan anti-HLA classe I, W6/32 [4]- suivi d'un contre-marquage avec le TO. L'analyse en cytométrie de flux a révélé que, comme observé chez la souris, les plaquettes TO^{bright} exprimaient des niveaux plus importants de molécules HLA I (Figure 3A, panneau de droite, surlignées en gris).

Pour analyser la distribution des molécules HLA I dans la population plaquettaire indépendemment de la taille des plaquettes, nous avons utilisé la même approche que pour les plaquettes murines, i.e. le ratio de la MFI HLA I/ paramètre FSC (Figure 3B). Encore une fois, nous avons trouvé que ce ratio était plus élevé dans les plaquettes TO^{bright} que dans les plaquettes TO^{dim} (ratio β2m/FSC 11.58 ± 1.25 dans TO^{bright} versus 6.59 ± 0.762 dans TO^{dim}, n=7, p< 0.05 et ratio W632/FSC 16.02 ± 1.99 dans TO^{bright} versus 8.46 ± 0.93 dans TO^{dim}, n=7, p< 0.001). A l'inverse, les ratios GPIbβ MFI/FSC étaient similaires entre les TO^{dim} et les TO^{bright} soulignant la particularité de l'expression des molécules HLA I sur les plaquettes jeunes.

### Les plaquettes humaines qui expriment fortement les molécules HLA I présentent un contenu en protéine ribosomale plus important, une caractéristique des plaquettes jeunes.

L'antigène de la protéine ribosomale P est présent sur la partie C-terminale de 3 sous unités ribosomales (RLP0, 2 et 3) qui sont nécessaires à la traduction des ARNm [5]. Puisque la traduction des ARNm se produit principalement dans les premières heures de vie d'une plaquette [2], on s'attendrait à détecter l'antigène de la protéine ribosomale P au moins dans les plaquettes TO^{bright}.

Les plaquettes humaines ont été fixées, perméabilisées et co-marquées avec l'anticorps dirigé contre l'antigène P de la protéine ribosomale, et un anticorps anti-β2m, un anticorps pan anti-HLA I, W6/32, et le TO avant d'être analysées par cytométrie de flux (Figure 4A).

Le marquage avec l'anticorps dirigé contre la protéine ribosomale P a révélé que 13.3± % (n=4) des plaquettes humaines exprimaient cette protéine alors que ce pourcentage était de 83 ± % (n=4) dans la sous population de plaquettes TO^{bright}, indiquant que cet antigène était plus exprimé par les plaquettes jeunes. Par ailleurs, l'analyse de l'expression des molécules du CMH I a mis en évidence une présence accrue sur les plaquettes TO^{bright} ou P-ribosomal- high en gris foncé (panneaux du bas). Nous avons ensuite analysé la distribution de la protéine ribosomale P dans les populations exprimant fortement ou faiblement les molécules HLA I (marquées avec W6/32 ou l'anti-β2m). Comme montré dans la Figure 4B, la protéine ribosomale P était exprimée dans la plupart des plaquettes HLA I^{high} mais seulement dans une minorité de plaquettes HLA I^{low} (82.77±10.63%, 10.47±3.59%, respectivement, n=4) (Figure 4B).

### Le niveau d'expression des molécules HLA I sur les plaquettes peut être utilisé comme critère pour évaluer la proportion de plaquettes jeunes chez un patient.

Pour étudier la pertinence de ces observations dans des applications cliniques, nous avons évalué l'expression des molécules HLA I à la surface des plaquettes humaines de donneurs sains ou de patients présentant des pathologies associées à une augmentation de la proportion de plaquettes jeunes dans la circulation. Nous avons d'abord comparé le pourcentage de plaquettes "HLA I^{high}" et des plaquettes TO^{bright} au pourcentage d'IPF donné par le Sysmex^{®} chez des volontaires sains (Figure 5A, n=6). Alors que le pourcentage de plaquettes HLA I^{high} et TO^{bright} étaient similaires (2.25% ± 0.26 vs 2.17 % ± 0.19, n=6), le pourcentage d'IPF s'est avéré plus hétérogène mais toujours inférieur à 15% chez les volontaires sains (6.7% ± 1.6, n=6).

Enfin, nous avons analysé l'expression des molécules HLA I à la surface des plaquettes de deux patients présentant un défaut au niveau de la myosine 9 et dont l'analyse au Sysmex^{®} montrait un fort pourcentage d'IPF (64.9 et 42.7 % d'IPF, respectivement).

Le sang citraté de chaque patient a été co-marqué avec un anticorps dirigé contre la β2m, un pan-anticorps anti HLA I, W6/32, et le TO avant d'être analysé par cytométrie de flux. De façon remarquable, seul un patient (P1) a présenté des ratios élevés de β2m/FSC et W6/32/FSC comparés au contrôle bien que le ratio TO/FSC soit plus important chez les deux patients (P1 and P2) comparé au volontaire sain (Figure 5B). En considérant le fait que le TO peut lier non seulement l'ARN mais aussi le contenu des granules, un traitement à la RNaseA a été effectué avant le marquage TO. Les analyses de cytométrie en flux ont montré que le traitement RNaseA a diminué de façon significative la proportion des plaquettes TO^{bright} dans le patient 1 (4.9% TO^{bright} après le traitement à la RNaseA vs 15.9% TO^{bright} avant le traitement à la RNaseA) alors que ce traitement n'affecte pas la population de plaquettes TO^{bright} dans le patient 2 (26.1 % TO^{bright} après le traitement à la RNaseA vs 29.4% TO^{bright} avant le traitement à la RNaseA) (Figure 5C). Puisque l'estimation des IPF est connue comme pouvant peut être biaisée par une activation des plaquettes, à cause notamment de la présence d'agrégats [6], nous avons vérifié si l'expression en surface des molécules HLA I pourrait être un critère plus fiable pour évaluer la proportion de plaquettes jeunes.

Du sang citraté a donc été incubé avec différentes concentrations d'un réactif d'agrégation plaquettaire, TRAP (0.1, 0.5, 1, 10, 50 ou 100 µM), avant d'être analysé par cytométrie de flux ou avec la technologie Sysmex^{®}. Alors que dès 0.5 µM de TRAP, une augmentation significative des IPF avec la technologie Sysmex^{®} a été constatée, (10.7±8% IPF pour la condition 0.5 µM TRAP vs 4.2±0.8 % d'IPF pour la condition non activée; n=3), les ratios β2m/FSC ou W632/FSC sont restés stables (1.3±0.4 dans la condition TRAP 0.5 µM versus 1.5±0.3 dans la condition non activée, n=3). Il a été noté qu'avec des doses plus élevées de TRAP (100µM), le ratio HLA I/FSC était augmenté, ce qui peut être dû à l'exposition à la membrane plasmatique des complexes HLA I présents dans les granules (Figure 5D). Cependant, il est intéressant de souligner que cette augmentation est restée comprise dans les valeurs de référence trouvées chez les volontaires sains.

Ces données suggèrent que le niveau d'expression des molécules HLA I est plus fiable que le marquage TO ou que la technologie Sysmex^{®} pour détecter des échantillons de patients présentant de fortes proportions de jeunes plaquettes.

### Intérêt de la normalisation avec FSC (FSC-A)

a) Des échantillons de sang de 7 donneurs sains et d'un patient présentant un syndrome de Bernard-Soulier ont été analysés avec le procédé selon l'invention, en calculant ou non un ratio avec FSC (i.e. avec et sans normalisation FSC). Les résultats sont présentés à la Figure 6.

Au regard des résultats obtenus sans normalisation, le patient BSS serait atteint d'une thrombopénie périphérique. En effet, les données sans normalisation semblent montrer que les plaquettes du patient BSS expriment plus de molécules HLA I à leur surface. Ce patient aurait donc plus de plaquettes jeunes que les donneurs sains.

Le calcul d'un ratio avec FSC montre que les plaquettes du patient BSS n'expriment pas plus de molécules HLA I à leur surface. Le patient BSS n'a donc pas plus de plaquettes jeunes que les donneurs sains. La normalisation permet donc d'éviter un faux positif.
b) Des échantillons de sang de 21 donneurs sains, d'un patient présentant une myélofibrose (MF), de deux patients présentant un syndrome de May-Hegglin (MYH9) et de deux patients présentant un purpura thrombotique thrombocytopénique (ITP) ont été analysés avec le procédé selon l'invention, en calculant ou non un ratio avec FSC (i.e. avec et sans normalisation FSC). Les résultats sont présentés à la Figure 7.

Sans rapporter à la taille des plaquettes, tous les patients seraient suspectés d'avoir plus de plaquettes jeunes et seraient donc étiquetés « thrombopénie périphérique ». Ce résultat est en accord avec la pathologie des patients MF et ITP (activité accrue de la moelle osseuse) alors que les patients MYH9 ne présentent pas forcément une activité accrue de la moelle.

Le ratio à la taille des plaquettes montre que le patient MYH9 2 est en fait un faux positif qui ne présente pas plus de plaquettes jeunes que les donneurs sains.

### Discussion

Les résultats ont montré que la mesure du niveau d'expression du CMH I en cytométrie de flux rapporté à la taille des plaquettes est utile pour analyser les plaquettes, en particulier pour identifier les plaquettes jeunes. Il a en effet été montré que les plaquettes jeunes expriment plus de CMH I à leur surface, ce niveau diminue avec l'âge de la plaquette. Tous ces résultats mettent en avant l'intérêt du ratio CMH I / FSC pour évaluer la proportion de plaquettes jeunes chez un sujet.

A ce jour, les cliniciens visualisent généralement le marquage de l'ARN avec le TO pour analyser les plaquettes jeunes présentes dans un échantillon (TO^{bright}). Mais cette technique présente des limites étant donné que le réactif utilisé pour lier l'ARN cytosolique (TO) peut aussi lier les nucléotides contenus dans les granules. La mesure de l'expression du CMH I permet d'éviter le marquage non-spécifique de l'ARN.

Par ailleurs, la mesure de l'expression de CMH I permet d'éviter un autre inconvénient, qui est la présence d'agrégats dans l'échantillon analysé, qui génère des faux positifs avec le procédé Sysmex^{®}.

Les résultats obtenus par les inventeurs ont été particulièrement précis lorsque la mesure du niveau d'expression du CMH I, en mesurant le paramètre MFI_{plaquettes}, a été normalisée avec le paramètre FSC permettent notamment de donner une indication sur la taille des particules, par exemple sur la taille des plaquettes puis avec l'intensité de fluorescence d'un standard interne (MFI_{standard}). Cette normalisation permet notamment de palier la variabilité inter-expérience, inter-laboratoire et/ou inter-expérimentateur.

Des résultats encore plus précis ont été obtenus lorsque que les inventeurs ont analysé les plaquettes en déterminant le ratio (MFI _{plaquettes} / FSC _{plaquettes}) / (MFI _{standard}/ FSC _{standard}) (i.e. MFI_{plaquettes normalisées} / FSC). Ce ratio permet d'analyser les plaquettes avec une finesse remarquable. Au final, cela permet de différencier plus finement une ou plusieurs population(s) de plaquette(s), en particulier une population de plaquettes jeunes. En conclusion, le niveau d'expression du CMH I mesuré en cytométrie de flux est un bon marqueur pour analyser les plaquettes présentes dans un échantillon sanguin, en particulier pour déterminer la proportion de plaquettes jeunes. La densité d'expression des molécules du CMH I constitue donc un marqueur de choix pour diagnostiquer une thrombopénie périphérique ou centrale chez un sujet et pour suivre l'efficacité thérapeutique d'un traitement de la thrombopénie centrale ou périphérique.

**Références** citées dans la demande sous la forme « [numéro de la référence] » :
1. Sun, K.H., et al., Monoclonal antibodies against human ribosomal P proteins penetrate into living cells and cause apoptosis of Jurkat T cells in culture. Rheumatology (Oxford), 2001. 40(7): p. 750-6.
2. Angenieux, C., et al., Time-Dependent Decay of mRNA and Ribosomal RNA during Platelet Aging and Its Correlation with Translation Activity. PLoS One, 2016. 11(1): p. e0148064.
3. Hechler, B., et al., Platelets are dispensable for antibody-mediated transfusion-related acute lung injury in the mouse. J Thromb Haemost, 2016. 14(6): p. 1255-67.
4. Tran, T.M., et al., The epitope recognized by pan-HLA class I reactive monoclonal antibody W6/32 and its relationship to unusual stability of the HLA-B27/beta2-microglobulin complex. Immunogenetics, 2001. 53(6): p. 440-6.
5. Derylo, K., et al., The uL10 protein, a component of the ribosomal P-statk, is released from the ribosome in nucleolar stress. Biochim Biophys Acta, 2018. 1865(1): p. 34-47.
6. Miyazaki, K., et al., Immature platelet fraction measurement is influenced by platelet size and is a useful parameter for discrimination of macrothrombocytopenia. Hematology, 2015. 20(10): p. 587-92.
7. Perrault, C. et al., Novel Monoclonal Antibody against the Extracellular Domain of GPIbModulates vWF Mediated Platelet Adhesion, Thromb Haemost, 2001. 86: p. 1238-48.
8. Nomura, S., Advances in Diagnosis and Treatments for Immune Thrombocytopenia, Clinical Medicine Insights: Blood Disorders, 2016. 9: p. 15-22.

## Revendications

1. Procédé pour identifier une population de plaquettes jeunes présente dans un échantillon sanguin, ledit procédé comprend les étapes de :
a) analyser les plaquettes présentes dans un échantillon sanguin par la mise en oeuvre d'un procédé comprenant les étapes de :
i. ajouter audit échantillon un ligand qui se lie au Complexe Majeur d'Histocompatibilité de classe I (CMH I) couplé à un fluorochrome ;
ii. mesurer l'intensité de fluorescence moyenne des plaquettes (MFI_{plaquettes}) avec un cytomètre en flux, et
iii. mesurer le paramètre « Forward Scatter » moyen (FSC) avec ledit cytomètre en flux et déterminer le ratio MFI_{plaquettes} / FSC ; et
b) utiliser le résultat de l'étape a) pour identifier une population de plaquettes jeunes.

2. Procédé selon la revendication 1, ledit échantillon sanguin est un échantillon de sang total ou une fraction du sang total comprenant les plaquettes.

3. Procédé selon l'une quelconque des revendications 1 ou 2, ledit ligand est un anticorps ou un fragment d'anticorps, par exemple ledit anticorps est l'anticorps W6/32 produit par l'hybridome ATCC HB-95 ou un dérivé qui a gardé sa capacité à lier le CMH I.

4. Procédé selon l'une quelconque des revendications 1 à 3, ledit fluorochrome est un fluorochrome dont les longueurs d'onde d'excitation et d'émission n'interfèrent pas avec les réactifs permettant de détecter l'ARN.

5. Procédé selon l'une quelconque des revendications 1 à 4, ladite mesure MFI_{plaquettes} est normalisée avec l'intensité de fluorescence moyenne d'un standard interne (MFI_{standard}) afin d'obtenir une intensité de fluorescence moyenne des plaquettes normalisée (MFI_{plaquettes normalisée}) et l'étape c) consiste à mesurer le paramètre « Forward Scatter » moyen (FSC) avec ledit cytomètre en flux pour déterminer le ratio MFI_{plaquettes} normalisée / FSC.

6. Procédé selon la revendication 5, ledit standard interne se lie audit ligand couplé au fluorochrome, par exemple le standard interne est une microbille de polystyrène qui se lie au ligand couplé au fluorochrome.

7. Procédé selon l'une quelconque des revendications 1 à 6, le ligand se lie au moins partiellement à la chaine alpha du CMH I.

8. Procédé de diagnostic *in vitro* d'une thrombopénie périphérique ou centrale chez un sujet comprenant les étapes de :
a) identifier une population de plaquettes jeunes présente dans un échantillon sanguin d'un sujet en mettant en oeuvre le procédé de l'une quelconque des revendications 1 à 7; et
b) utiliser le résultat de l'étape a) dans le diagnostic de la thrombopénie périphérique ou centrale.

9. Procédé selon la revendication 8, dans lequel un diagnostic positif pour la thrombopénie périphérique est donné par une augmentation chez ledit sujet du ratio MFI_{plaquettes} / FSC et/ou du ratio MFI_{plaquettes normalisée} / FSC par rapport au(x) même(s) paramètre(s) mesuré(s) chez un sujet sain.

10. Procédé selon la revendication 8, dans lequel un diagnostic positif pour la thrombopénie centrale est donné par un/des paramètre(s) ratio MFI_{plaquettes} / FSC et/ou ratio MFI_{plaquettes normalisée} / FSC chez ledit sujet similaire(s) ou identique(s) au(x) même(s) paramètre(s) mesuré(s) chez un sujet sain.

11. Procédé *in vitro* pour pronostiquer le développement d'une maladie inflammatoire ou cardiovasculaire, comprenant les étapes de :
a) identifier une population de plaquettes jeunes présente dans un échantillon sanguin d'un sujet en mettant en oeuvre le procédé de l'une quelconque des revendications 1 à 7 ;
b) utiliser le résultat de l'étape a) dans le pronostic du développement de la maladie inflammatoire ou cardiovasculaire.

12. Procédé *in vitro* de suivi de l'efficacité thérapeutique d'un traitement de la thrombopénie centrale ou périphérique, comprenant les étapes de :
a) identifier une population de plaquettes jeunes présente dans un échantillon sanguin d'un patient ayant subi un traitement de la thrombopénie centrale ou périphérique en mettant en oeuvre le procédé d'analyse selon l'une quelconque des revendications 1 à 7 ;
b) utiliser le résultat de l'étape a) dans la détermination de l'efficacité thérapeutique du traitement de la thrombopénie centrale ou périphérique.

## Patentansprüche

1. Verfahren zum Identifizieren einer Population an jungen Blutplättchen, die in einer Blutprobe vorhanden ist, wobei das Verfahren die Schritte umfasst des:
a) Analysierens der Blutplättchen, die in einer Blutprobe vorhanden sind, durch Ausführen eines Verfahrens, das die Schritte umfasst des:
i. Hinzufügens eines Liganden zu der Probe, der an den Haupthistokompatibilitätskomplex Klasse I (HHK I) bindet und der an ein Fluorochrom gekoppelt ist,
ii. Messens der mittleren Fluoreszenzintensität der Blutplättchen (MFI_{Blutplättchen}) mit einem Durchflusszytometer, und
iii. Messens des Parameters des mittleren "Forward Scatter" (FSC) mit dem Durchflusszytometer und Bestimmens des Verhältnisses MFI_{Blutplättchen}/FSC, und
b) Verwendens des Ergebnisses aus Schritt a), um eine Population an jungen Blutplättchen zu identifizieren.

2. Verfahren nach Anspruch 1, wobei die Blutprobe eine Vollblutprobe oder ein Anteil des Vollblutes ist, der Blutplättchen umfasst.

3. Verfahren nach einem der Ansprüche 1 oder 2, wobei der Ligand ein Antikörper oder ein Antikörperfragment ist, zum Beispiel der Antikörper der Antikörper W6/32, der durch das Hybridom ATCC HB-95 erzeugt wird, oder ein Derivat ist, das seine Fähigkeit bewahrt hat, an den HHK I zu binden.

4. Verfahren nach einem der Ansprüche 1 bis 3, wobei das Fluorochrom ein Fluorochrom ist, dessen Anregungs- und Emissionswellenlängen die Reagenzien zum Nachweisen von RNA nicht stören.

5. Verfahren nach einem der Ansprüche 1 bis 4, wobei die Messung der MFI_{Blutplättchen} auf die mittlere Fluoreszenzintensität eines internen Standards (MFI_{standard}) normiert wird, um eine normierte mittlere Fluoreszenzintensität der Blutplättchen (MFI_{Blutplättchen normiert}) zu erhalten, und der Schritt c) aus Messen des Parameters des mittleren "Forward Scatter" (FSC) mit dem Durchflusszytometer besteht, um das Verhältnis MFI_{Blutplättchen normiert}/FSC zu bestimmen.

6. Verfahren nach Anspruch 5, wobei der interne Standard an den Liganden bindet, der an das Fluorochrom gekoppelt ist, zum Beispiel der interne Standard ein Mikrokügelchen aus Polystyrol ist, das an den Liganden bindet, der an das Fluorochrom gekoppelt ist.

7. Verfahren nach einem der Ansprüche 1 bis 6, wobei der Ligand zumindest teilweise an die alpha-Kette des HHK I bindet.

8. In-vitro-Diagnoseverfahren für eine periphere oder zentrale Thrombozytopenie bei einem Individuum, umfassend die Schritte des:
a) Identifizierens einer Population an jungen Blutplättchen, die in einer Blutprobe eines Individuums vorhanden ist, durch Ausführen des Verfahrens nach einem der Ansprüche 1 bis 7, und
b) Verwendens des Ergebnisses aus Schritt a) bei der Diagnose der peripheren oder zentralen Thrombozytopenie.

9. Verfahren nach Anspruch 8, wobei eine Positivdiagnose für die periphere Thrombozytopenie durch eine Erhöhung des Verhältnisses MFI_{Blutplättchen}/FSC und/oder des Verhältnisses MFI_{Blutplättchen normiert}/FSC in Bezug auf den/die gleiche(n) bei einem gesunden Individuum gemessene(n) Parameter bei dem Individuum gegeben ist.

10. Verfahren nach Anspruch 8, wobei eine Positivdiagnose für die zentrale Thrombozytopenie dadurch gegeben ist, dass ein/die Parameter des Verhältnisses MFI_{Blutplättchen}/FSC und/oder des Verhältnisses MFI_{Blutplättchen normiert}/FSC bei dem Individuum ähnlich oder identisch mit dem/den gleichen bei einem gesunden Individuum gemessenen Parameter(n) ist/sind.

11. In-vitro-Verfahren zum Prognostizieren der Entwicklung einer entzündlichen oder kardiovaskulären Erkrankung, umfassend die Schritte des:
a) Identifizierens einer Population an jungen Blutplättchen, die in einer Blutprobe eines Individuums vorhanden ist, durch Ausführen des Verfahrens nach einem der Ansprüche 1 bis 7,
b) Verwendens des Ergebnisses aus Schritt a) bei der Prognose der Entwicklung der entzündlichen oder kardiovaskulären Erkrankung.

12. In-vitro-Verfahren zum Verfolgen der therapeutischen Wirksamkeit einer Behandlung für zentrale oder periphere Thrombozytopenie, umfassend die Schritte des:
a) Identifizierens einer Population an jungen Blutplättchen, die in einer Blutprobe eines Patienten vorhanden ist, der sich einer Behandlung für zentrale oder periphere Thrombozytopenie unterzogen hat, durch Ausführen des Analyseverfahrens nach einem der Ansprüche 1 bis 7,
b) Verwendens des Ergebnisses aus Schritt a) bei der Bestimmung der therapeutischen Wirksamkeit der Behandlung für zentrale oder periphere Thrombozytopenie.

## Claims

1. A process for identifying a population of young platelets present in a blood sample, said process comprises the steps of:
a) analyzing platelets present in a blood sample by carrying out a comprising the steps of:
i. adding to said sample a ligand that binds to Major Histocompatibility Complex I (MHC I) coupled to a fluorochrome;
ii. measuring the mean fluorescence intensity of the platelets (MFIₚₗₐₜₑₗₑₜₛ) with a flow cytometer; and
iii. measuring the mean forward scatter (FSC) parameter with said flow cytometer and determining the MFIₚₗₐₜₑₗₑₜₛ / FSC ratio; and
b) using the result of step a) to identify a population of young platelets.

2. The process as claimed in claim 1, said blood sample is a sample of whole blood or a fraction of whole blood including platelets.

3. The process as claimed in any one of claims 1 or 2, said ligand is an antibody or an antibody fragment, for example said antibody is the W6/32 antibody produced by the hybridoma ATCC HB-95 or a derivative which has retained its ability to bind MHC I.

4. The process as claimed in any one of claims 1 to 3, said fluorochrome is a fluorochrome the excitation and emission wavelengths of which do not interfere with reagents for detecting RNA.

5. The process as claimed in any one of claims 1 to 4, said MFIₚₗₐₜₑₗₑₜₛ measurement is normalized to the mean fluorescence intensity of an internal standard (MFI_{standard}) in order to obtain a normalized platelet mean fluorescence intensity (MFI_{normalized platelets}) and step c) consists in measuring the mean forward scatter (FSC) parameter with said flow cytometer to determine the MFI_{normalized platelets/} FSC ratio.

6. The process as claimed in claim 5, said internal standard binds to said fluorochrome-coupled ligand, for example the internal standard is a polystyrene microbead that binds to the fluorochrome-coupled ligand.

7. The process as claimed in any one of claims 1 to 6, the ligand binds at least partially to the MHC I alpha chain.

8. A process for the *in vitro* diagnosis of peripheral or central thrombocytopenia in a subject comprising the steps of:
a) identifying a population of young platelets present in a blood sample of a subject by carrying out the process of any one of claims 1 to 7; and
b) using the result of step a) in the diagnosis of peripheral or central thrombocytopenia.

9. The process as claimed in claim 8, wherein a positive diagnosis for peripheral thrombocytopenia is given by an increase in said subject in the MFIₚₗₐₜₑₗₑₜₛ / FSC ratio and/or the MFI_{normalized platelets} / FSC ratio relative to the same parameter(s) measured in a healthy subject.

10. The process as claimed in claim 8, wherein a positive diagnosis for central thrombocytopenia is given by parameter(s) MFIₚₗₐₜₑₗₑₜₛ / FSC ratio and/or MFI_{normalized platelets} / FSC ratio in said subject similar or identical to the same parameter(s) measured in a healthy subject.

11. An *in vitro* process for prognosing the development of an inflammatory or cardiovascular disease, comprising the steps of:
a) identifying a population of young platelets present in a blood sample from a subject by carrying out the process as claimed any one of claims 1 to 7;
b) using the result of step a) in the prognosis of the development of inflammatory or cardiovascular disease.

12. An *in vitro* process for monitoring the therapeutic efficacy of a treatment for central or peripheral thrombocytopenia, comprising the steps of:
a) identifying a population of young platelets present in a blood sample of a patient having undergone central or peripheral thrombocytopenia treatment by carrying out the analysis process as claimed in any one of claims 1 to 7;
b) using the result of step a) in determining the therapeutic efficacy of the treatment of central or peripheral thrombocytopenia.
